# EUROPÄISCHE PATENTSCHRIFT

(11) **EP 1 534 740 B1**
(45) Veröffentlichungstag und Bekanntmachung des Hinweises auf die Patenterteilung: **27.12.2006**
(21) Anmeldenummer: 03762452.5
(22) Anmeldetag: 04.07.2003
(51) Int. Cl.: C07K 14/00, C07K 17/00, G01N 33/68

(54) **VERFAHREN ZUR STABILISIERUNG VOM BIOMOLEKÜLEN**
METHOD FOR STABILISING BIOMOLECULES
PROCEDE DE STABILISATION DE BIOMOLECULES

(30) Priorität: 04.07.2002 DE 10230210
(43) Veröffentlichungstag der Anmeldung: 01.06.2005
(73) Patentinhaber: Micronas Holding GmbH, 79108 Freiburg (DE); Klapproth, Holger, Dr., 79108 Freiburg (DE)
(72) Erfinder: KLAPPROTH, Holger, 79108 Freiburg (DE)
(74) Vertreter: Stürken, Joachim
(86) Internationale Anmeldenummer: PCT/DE2003/002245
(87) Internationale Veröffentlichungsnummer: WO 2004/004455

(56) Entgegenhaltungen:
- US-A- 5 624 831
- WOLKERS WF ET AL.: "Isolation and characterization of a D-7 LEA protein from pollen that stabilizes glasses in vitro" BIOCHIMICA ET BIOPHYSICA ACTA, Bd. 1544, 2001, Seiten 196-206, XP002265093
- SALES K ET AL.: "The LEA-like protein HSP 12 in Saccharomyces cerevisiae has a plasma membrane location and protects membranes against desiccation and ethanol-induced stress" BIOCHIMICA ET BIOPHYSICA ACTA, Bd. 1463, 2000, Seiten 267-278, XP002265094
- BROWNE J ET AL.: "Plant desiccation gene found in a nematode" NATURE, Bd. 416, 7. März 2002 (2002-03-07), Seite 38 XP002265095 in der Anmeldung erwähnt

### SEQUENZPROTOKOLL

<110> Micronas Holding GmbH
   Klapproth, Holger
<120> Zusammensetzung und Verfahren zur Stabilisierung von Biomolekülen
<130> 1034
<140>
   <141>
<160> 9
<170> PatentIn Ver. 2.1
<210> 1
   <211> 11
   <212> PRT
   <213> Künstliche Sequenz
<220>
   <223> Beschreibung der künstlichen Sequenz: Motiv der LEA Klasse
<220>
   <223> Aminosäureposition 1: X= K oder T
<220>
   <223> Aminosäureposition 2: X=A oder G oder K oder M oder T
<220>
   <223> Aminosäureposition 3: X=R oder D oder A oder E oder Q oder K
<220>
   <223> Aminosäureposition 4: X= E oder K oder S
<220>
   <223> Aminosäureposition 5: X= T oder F oder Y oder A
<220>
   <223> Aminosäureposition 6: X= K oder R oder T oder A
<220>
   <223> Aminosäureposition 7: X= D oder E oder Q
<220>
   <223> Aminosäureposition 8: X= S oder R oder Y oder K
<220>
   <223> Aminosäureposition 9: X= A oder T
<220>
   <223> Aminosäureposition 10: X= G oder A oder R
<400> 1
   Xaa Xaa Xaa Xaa Xaa Xaa Xaa Xaa Xaa Xaa Glu
   1               5                   10
<210> 2
<211> 11
   <212> PRT
   <213> Aphelenchus avenae
<400> 2
   Lys Thr Ala Glu Phe Arg Asp Ser Ala Gly Glu
   1 5 10
<210> 3
   <211> 11
   <212> PRT
   <213> Aphelenchus avenae
<400> 3
   Lys Gly Gln Glu Phe Lys Glu Arg Ala Gly Glu
   1 5 10
<210> 4
   <211> 11
   <212> PRT
   <213> Aphelenchus avenae
<400> 4
   Lys Ala Glu Glu Thr Lys Gln Arg Ala Gly Glu
   1 5 10
<210> 5
   <211> 11
   <212> PRT
   <213> Aphelenchus avenae
<400> 5
   Lys Met Asp Glu Thr Lys Gln Arg Ala Gly Glu
   1 5 10
<210> 6
   <211> 11
   <212> PRT
   <213> Betula pendula
<400> 6
   Lys Ala Arg Lys Thr Lys Asp Ser Ala Ala Glu
   1 5 10
<210> 7
   <211> 11
   <212> PRT
   <213> Betula pendula
<400> 7
   Lys Ala Lys Glu Tyr Lys Asp Tyr Thr Ala Glu
   1 5 10
<210> 8
   <211> 11
   <212> PRT
   <213> Betula pendula
<400> 8
   Lys Ala Arg Glu Thr Thr Glu Lys Ala Arg Glu
   1 5 10
<210> 9
   <211> 11
   <212> PRT
   <213> Betula pendula
<400> 9
   Thr Lys Asp Ser Ala Ala Glu Lys Ala Arg Glu
   1 5 10

## Beschreibung

Die vorliegende Erfindung betrifft allgemein das Gebiet der Biotechnologie. Insbesondere betrifft die Erfindung Verfahren und Zusammensetzungen zur Stabilisierung bzw. Konservierung von biologischen Molekülen sowie Vorrichtungen, welche entsprechend stabilisierte Biomoleküle aufweisen.

Die Verwendung von Proteinen und Polypeptiden in industriellen Produkten und Verfahren erfordert große Mengen dieser Biomoleküle, insbesondere für klinisch-diagnostische und pharmazeutische Zwecke. Während die hierfür erforderlichen Basistechniken wie Isolierung und Aufreinigung von Proteinen im industriellen Maßstab zumeist etabliert sind, liegt der schwierigste Aspekt der Verwendung derartiger Biomoleküle in der Aufrechterhaltung der für die beabsichtigte Verwendung gewünschten nativen bzw. aktiven Moleküleigenschaften. Insbesondere bei der Lagerung und dem Transport von Biomolekülen müssen häufig Verluste der Aktivität hingenommen werden, wodurch der Erfolg späterer Anwendungen gefährdet sein kann. Kommerziell erhältliche Proteinpräparationen enthalten daher zumeist Verbindungen, durch deren Anwesenheit der Aktivitätsverlust während der Lagerung oder des Transports vermindert werden kann. Weiterhin erfolgen die Lagerung sowie der Transport zumeist unter niedrigen Temperaturen, obgleich ein Einfrieren von z.B. bestimmten Proteinen aufgrund molekularer Veränderungen unerwünscht sein kann.

Aus der Literatur ist bekannt, dass bestimmte Pflanzen und Tiere Mechanismen entwickelt haben, um den Zustand einer annähernd vollständigen Dehydratation zu überleben. Dieser Stresszustand wird als Anhydrobiose bezeichnet und wird bei Organismen beobachtet, die Trockenbedingungen ausgesetzt werden. Während der Anhydrobiose befindet sich der Organismus in einer Art Ruhezustand, bis eine Rehydratation es erlaubt, den normalen Stoffwechsel fortzusetzen. Gemeinsame, charakteristische Eigenschaft dieser Organismen ist die Synthese von hohen Konzentrationen an nicht-reduzierenden Zuckern, die durch anhydrobiotische Bedingungen induziert wird. Die als Antwort auf Dehydratisierung bei verschiedenen Organismen beobachtete Akkumulation großer Mengen an Trehalose führt zu einem Schutz von Membranen und Proteinen vor Schädigungen der molekularen Integrität und korreliert mit einer gewissen Toleranz gegenüber Wasserentzug. Man nimmt an, dass der Zucker die entzogenen Wassermoleküle ersetzt bzw. funktional substituiert und an der Bildung eines intrazellulären organischen Glases beteiligt ist, von dem man vermutet, dass es die Zellinhalte stabilisiert.

Im Stand der Technik wird die Verwendung von Trehalose bei der Herstellung von mit Antikörpern beschichteten Mikrotiterplatten beschrieben, um diese normalerweise sehr schnell denaturierenden Proteinspecies zu stabilisieren (V. K. Nguyen et al., Protection of immunoreactivity of dry immobilized proteins on microtitration plates in ELISA: application for detection of autoantibodies in *Myasthenia gravis, J. of Biotechnology,* 72, S. 115 bis 125 (1999 ; US-A-5 624 831). Hierzu wurden Mikrotiterplatten mit darauf immobilisierten Antikörpern mit einem Rinderserumalbumin (BSA) und Trehalose enthaltenden Film beaufschlagt und anschließend getrocknet. Die auf den in dieser Weise erzeugten ELISA-Platten immobilisierten, getrockneten Antikörper zeigten auch bei erhöhten Temperaturen (bis zu 50 °C) eine Lagerungsfähigkeit von bis zu 30 Tagen.

Eine insbesondere aus wirtschaftlicher Sicht anzustrebende Verlängerung der Lagerungsfähigkeit unter Raumtemperatur- oder sogar Tropenbedingungen kann mit dieser Technologie jedoch nicht erreicht werden. Insbesondere im Zusammenhang mit der Bereitstellung großer Mengen an immobilisierten Proteinen ist eine Lagerungsfähigkeit für eine Zeitdauer von über einem Jahr bei im wesentlichen gleichbleibender biologischer Aktivität bzw. Funktionalität der Biomoleküle wünschenswert.

Ein weiterer Nachteil der zuvor beschriebenen Technologie liegt in der Verwendung von Rinderserumalbumin (BSA), einem Proteingemisch, von dem bekannt ist, dass es im Rahmen Antikörper-gestützter Anwendungen unspezifische Bindungen mit den Antikörpern eingeht und damit unerwünschte Kreuzreaktionen verursacht, durch die das gesamte Untersuchungsergebnis negativ beeinträchtigt wird.

Insbesondere von pflanzlichen Samen und Pollen ist bekannt, dass in ihnen als Reaktion auf Wasserentzug Proteine der LEA-Klasse gebildet werden (LEA = 'late embryogenesis abundant')(J. Ingram und D. Bartels, *Annu. Rev. Plant Physiol. Plant Mol. Biol.,* 47, S. 377 bis 403 (1996)). Die mittlerweile auch in Nematoden identifizierten LEA-Proteine enthalten ein spezielles, 11 Aminosäuren umfassendes Motiv, welches vermutlich eine amphipatische α-Helix ausbildet, durch das die Oligomerisierung des Proteins gesteuert wird. Die LEA-Proteine sind extrem hydrophil und gegenüber einer Denaturierung durch Hitze resistent. Erste Versuche mit einem aus Pollen von. *Typha latifolia* gereinigten Protein dieser Klasse haben gezeigt, dass sich Saccharosegläser in vitro durch Inkubation mit diesem Protein stabilisieren lassen (W.F. Wolkers et al., "Isolation and characterization of a D-7 LEA protein from pollen that stabilizes glasses in vitro", Biochimica et Biophysica Acta, Bd. 1544, 2001, Seiten 196 - 206). Es wird daher vermutet, dass nichtreduzierende Zucker und Vertreter der LEA-Proteinklasse in synergistischer Weise zusammenwirken bei der Bildung eines stabilen Bioglases im Cytoplasma von anhydrobiotischen Pflanzen und in gegenüber Austrocknung resistenten Samen und Pollen (J. Brown et al., Plant desiccation gene found in a nematode, Nature, 416, S. 38 (2002)). Ferner wurde in den Plasmamembranen von *Saccharomyces cerevisiae* das LEA-ähnliche Protein HSP 12 identifiziert (K. Sales et al., Biochim. et Biophys. Acta, Bd. 1463, 2000, S. 267-278), welches die Membranen vor der Austrocknung und Ethanol-induziertem Stress schützt. In einem Membran-Modellsystem zeigte sich in vitro, dass sich HSP 12 analog zu Trehalose verhielt und die Integrität der Membran gegenüber Austrocknung schützte.

Aufgabe der Erfindung ist daher die Bereitstellung von Verfahren und Zusammensetzungen zur Stabilisierung bzw. Konservierung von Biomolekülen, mit denen die Nachteile des Standes der Technik überwunden werden und mit denen die gewünschte biologische Aktivität der Moleküle über einen längeren Zeitraum auch ohne Kühlung gewährleistet wird. Die Aufgabe wird erfindungsgemäß durch das Verfahren gemäß Hauptanspruch gelöst.

Die für das erfindungsgemäße Verfahren verwendete Zusammensetzung umfasst mindestens ein nicht-reduzierendes Disaccharid, ausgewählt aus der Gruppe bestehend aus Trehalose (D-Glucopyranosyl-D-glucopyranose) und Saccharose (β-D-Fructofuranosyl-α-D-glycopyranosid), und mindestens ein Protein oder Polypeptid der LEA-Klasse. Nach einer bevorzugten Ausführungsform umfasst die Zusammensetzung Trehalose sowie mindestens ein Protein oder Polypeptid der LEA-Klasse mit einem 11 Aminosäuren umfassenden Motiv, welches durch die folgende allgemeine Formel (SEQ ID NO. 1) charakterisiert ist:
(1)-(2)_{`}(3)-(4)-(5)-(6)-(7)-(8)-(9)-(10)-E.
wobei (1) K oder T,
(2) A, G, K, M oder T,
(3) R, D, A, E, Q oder K,
(4) E, K oder S,
(5) T, F, Y oder A,
(6) K, R, T oder A,
(7) D, E oder Q,
(8) S, R, Y oder K,
(9) A oder T, und
(10) G, A oder R,
bedeuten. In einer besonders bevorzugten Ausführungsform umfasst die Zusammensetzung mindestens ein Protein oder Polypeptid der LEA-Unterklasse 3 mit einer Aminosäuresequenz, die von einer Nukleotidsequenz kodiert wird, wie sie in der GenBank unter der Zugriffsnummer AF423069 oder S39475 hinterlegt ist. Die für das erfindungsgemäße Verfahren vorgeschlagene Zusammensetzung umfasst am meisten bevorzugt mindestens ein Protein oder Polypeptid der LEA-Unterklasse 3 mit einem 11 Aminosäuren umfassenden Motiv, ausgewählt aus der Gruppe bestehend aus:
(a) K-T-A-E-F-R-D-S-A-G-E (SEQ ID NO. 2),
(b) K-G-Q-E-F-K-E-R-A-G-E (SEQ ID NO. 3),
(c) K-A-E-E-T-K-Q-R-A-G-E (SEQ ID NO. 4),
(d) K-M-D-E-T-K-Q-R-A-G-E (SEQ ID NO. 5),
(e) K-A-R-K-T-K-D-S-A-A-E (SEQ ID NO. 6),
(f) K-A-K-E-Y-K-D-Y-T-A-E (SEQ ID NO. 7),
(g) K-A-R-E-T-T-E-K-A-R-E (SEQ ID NO. 8), und
(h) T-K-D-S-A-A-E-K-A-R-E (SEQ ID NO. 9).

Nach einer bevorzugten Ausführungsform wird eine Zusammensetzung verwendet, welche die Komponenten des nicht-reduzierenden Disaccharids und des Proteins oder Polypeptids der LEA-Klasse in jeweiligen Mengen von 0,01 bis 15 beziehungsweise 0,00001 bis 1 Gewichtsprozent, jeweils bezogen auf eine gebrauchsfertige Lösung, umfasst. Beispielsweise wird die Zusammensetzung hergestellt, indem einer Lösung aus 50 mM Phosphat und 100 mM NaCl mit einem pH-Wert von 6,8 0,1 Gew.% gereinigtes LEA-Protein und 5 Gew.% Trehalose zugegeben werden. Gewünschtenfalls können auch weitere Komponenten wie z.B. Natriumazid (0,02 Gew.%) zugesetzt werden.

Für den Fachmann ist klar, dass er anhand der erfindungsgemäß bereitgestellten Sequenz- und Motivinformationen homologe Vertreter der Proteinklasse LEA aus anderen Quellen auffinden und erfindungsgemäß einsetzen kann. Folglich sind alle Homologa umfasst, sofern sie in der Lage sind, ein aus nicht-reduzierenden Zuckern wie insbesondere Trehalose und/oder Saccharose gebildetes biologisches Glas zu stabilisieren.

Beispielhafte Vertreter der Proteinklasse LEA sind in der nachfolgenden Tabelle aufgeführt, stellen aber keine Beschränkung der vorliegenden Erfindung dar. Die jeweiligen Sequenzinformationen können vom Fachmann anhand der bereitgestellten Daten leicht erhalten werden.

| Datenbank | Organismus | Bezeichnung (Zugriffsnummer) |
|---|---|---|
| SWISS-PROT | Gossypium hirsutum | P09422 |
| SWISS-PROT | Raphanus sativus | P21208 |
| SWISS-PROT | Zea mays | P46517 |
| SWISS-PROT | Hordeum vulgare | Q02400 |
| SWISS-PROT | Hordeum vulgare | Q05191 |
| SWISS-PROT | Hordeum vulgare | Q5190 |
| SWISS-PROT | Hordeum vulgare | P46532 |
| SWISS-PROT | Helianthus annuus | P46515 |
| SWISS-PROT | Helianthus annuus | P46515 |
| SWISS-PROT | Gossypium hirsutum | P09411 |
| SWISS-PROT | Gossypium hirsutum | P46518 |
| SWISS-PROT | Gossypium hirsutum | P09443 |
| SWISS-PROT | Gossypium hirsutum | P13940 |
| SWISS-PROT | Gossypium hirsutum | P09444 |
| SWISS-PROT | Gossypium hirsutum | P46521 |
| SWISS-PROT | Gossypium hirsutum | P4522 |
| SWISS-PROT | Brassica napus | P13934 |
| SWISS-PROT | Gossypium hirsutum | P13939 |
| SWISS-PROT | Zea mays | Q42376 |
| SWISS-PROT | Tricitum aestivum | Q03968 |
| TrEMBL | Phaseolus vulgaris | 024442 |
| TrEMBL | Arabidopsis thaliana | 064820 |
| TrEMBL | Arabidopsis thaliana | 065148 |
| TrEMBL | Arabidopsis thaliana | 080576 |
| TrEMBL | Arabidopsis thaliana | 081483 |
| TrEMBL | Oryza sativa | P83196 |
| TrEMBL | Oryza sativa | P83197 |
| TrEMBL | Glycine max | P93165 |
| TrEMBL | Glycine max | Q01527 |
| TrEMBL | Tricitum aestivum | Q03967 |
| TrEMBL | Arabidopsis thaliana | Q39138 |
| TrEMBL | Citrus sinensis | Q39466 |
| TrEMBL | Chlorella vulgaris | Q39660 |
| TrEMBL | Gossypium hirsutum | Q39793 |
| TrEMBL | Gossypium hirsutum | Q39797 |
| TrEMBL | Glycine soja | Q39919 |
| TrEMBL | Onoclea sensibilis | Q40697 |
| TrEMBL | Picea glauca | Q40842 |
| TrEMBL | Picea glauca | Q40843 |
| TrEMBL | Picea glauca | Q40848 |
| TrEMBL | Picea glauca | Q40858 |
| TrEMBL | Picea glauca | Q40869 |
| TrEMBL | Zea mays | Q41804 |
| TrEMBL | Oryza sativa | Q8S4X7 |
| TrEMBL | Brassica campestris | Q8S8Z2 |
| TrEMBL | Brassica napus | Q8S8Z2 |
| TrEMBL | Arabidopsis thaliana | Q96273 |
| TrEMBL | Oryza sativa | Q9AWZ5 |
| TrEMBL | Arabidopsis thaliana | Q9FG31 |
| TrEMBL | Arabidopsis thaliana | Q9FK14 |
| TrEMBL | Arabidopsis thaliana | Q9FK15 |
| TrEMBL | Arabidopsis thaliana | Q9FKV7 |
| TrEMBL | Oryza sativa | Q9FPB2 |
| TrEMBL | Arabidopsis thaliana | Q9LF88 |
| TrEMBL | Oryza sativa | Q9LGL8 |
| TrEMBL | Arabidopsis thaliana | Q9LT74 |
| TrEMBL | Euphorbia esula | Q9M556 |
| TrEMBL | Arabidopsis thaliana | Q9S7S3 |
| TrEMBL | Oncolea sensibilis | Q9S2B2 |
| TrEMBL | Arabidopsis thaliana | Q9SKP0 |
| TrEMBL | Chlorella vulgaris | Q9SLP7 |
| TrEMBL | Arabidopsis thaliana | Q9XID7 |
| TrEMBL | Arabidopsis thaliana | Q9ZPW6 |
| TrEMBL | Glycine max | Q9ZTZ2 |

Die erfindungsgemäß vorgeschlagenen LEA-Proteine oder Polypeptide können aus natürlichen Quellen isoliert, rekombinant hergestellt oder synthetisiert werden. Die hierfür anzuwendenden Verfahren sind dem Fachmann gut bekannt.

Ein weiterer Aspekt der vorliegenden Erfindung betrifft die Bereitstellung eines Verfahrens zur Stabilisierung bzw. Konservierung von Biomolekülen, bei dem die zu schützenden Moleküle in der erfindungsgemäßen Zusammensetzung inkubiert werden. Nach ausreichender Inkubationsdauer kann der Ansatz nachfolgend z.B. bei Raumtemperatur trocknen und bis zur Verwendung ohne Kühlung gelagert werden. Sofern das Verfahren zur Stabilisierung von auf bestimmten Oberflächen immobilisierten Biomolekülen angewendet werden soll, werden diese beladenen Oberflächen mit der erfindungsgemäßen Zusammensetzung beaufschlagt. Dies kann beispielsweise durch Aufsprühen der Zusammensetzung auf die Oberfläche oder durch Eintauchen der Oberfläche in die Zusammensetzung erfolgen. Im Falle des Tauchverfahrens sollte die Oberfläche vorzugsweise mit einer Geschwindigkeit von etwa 2 mm pro Sekunde herausgezogen werden, damit eine gleichmäßige Benetzung mit der erfindungsgemäßen Zusammensetzung erfolgen kann.

Ein weiterer Aspekt der vorliegenden Erfindung betrifft die Bereitstellung von Oberflächen, die mit der erfindungsgemäßen Zusammensetzung beaufschlagt sind. Auf bevorzugten Oberflächen sind Biomoleküle direkt oder indirekt immobilisiert und werden von der erfindungsgemäßen Zusammensetzung stabilisiert bzw. konserviert. Besonders bevorzugte Oberflächen sind Bestandteile von analytischen und/oder diagnostischen Vorrichtungen, wie z.B. Biochips, Sensorchips, Mikrotiterplatten, Reaktionsröhrchen und dergleichen. Das Material der Oberfläche unterliegt hierbei keiner Beschränkung und kann beispielsweise aus Glas, Quarzglas, Quarz, Silizium, Polymeren (PMMA, Polystyrol, Polyethylen, Polypropylen, PVC etc.), und Membranen wie z.B. Nitrozellulose-, Nylon- und Mikrofasermembranen sowie Papier ausgewählt werden.

Die vorliegend verwendeten Begriffe "biologische Moleküle" und "Biomoleküle" umfassen jedwede Substanzen und Verbindungen im wesentlichen biologischen Ursprungs, die über Eigenschaften verfügen, welche im Rahmen wissenschaftlicher, diagnostischer und/oder pharmazeutischer Anwendungen relevant sind. Umfasst sind nicht nur native Moleküle, wie sie aus natürlichen Quellen isoliert werden können, sondern auch davon abgeleitete Formen, Fragmente und Derivate, sowie rekombinante Formen und artifizielle Moleküle, sofern sie mindestens eine Eigenschaft der nativen Moleküle aufweisen. Bevorzugte Biomoleküle sind solche, die für analytische, diagnostische und/oder pharmazeutische Zwecke eingesetzt werden können, wie Nukleinsäuren und deren Derivate (DNA, RNA, PNA, LNA, Ribozyme, Oligonukleotide, Plasmide, Chromosomen), Peptide und Proteine (Enzyme, Rezeptorproteine, Proteinkomplexe, Peptidhormone, Antikörper) sowie biologisch aktive Fragmente derselben, Kohlenhydrate und deren Derivate wie insbesondere glykosylierte Proteine und Glykoside, und Fette, Fettsäuren und Lipide.

Für den Fachmann ist klar, dass sich die erfindungsgemäßen Zusammensetzungen und Verfahren auch auf zelluläre Gewebe und vollständige Zellen sowie Teile derselben (Organellen, Membranen und Membranfragmente etc.) anwenden lassen, sofern sie Träger der obigen Biomoleküle sind. Deshalb sind Gewebe, Zellen und Teile derselben grundsätzlich vom Begriff "Biomoleküle" umfasst.

Die verwendeten Begriffe "Stabilisierung" und "Konservierung" beziehen sich auf die strukturelle bzw. funktionelle Integrität von Biomolekülen und den hierauf basierenden biologischen Eigenschaften. Die für einen bestimmten Anwendungszweck erforderliche Aktivität eines Biomoleküls erfordert z.B. die weitgehende Beibehaltung seiner Primär-, Sekundär- und/oder Tertiärstruktur. Die biologische Aktivität einer Nukleinsäuresonde umfasst beispielsweise ihre Eigenschaft zur Ausbildung eines Hybridisierungskomplexes mit einem Nukleinsäuretarget, welches zur Sonde komplementär ist. Die biologische Aktivität eines Antikörpers umfasst beispielsweise die spezifische Bindung seines Antigens.

Die Erfindung wird anhand des nachfolgenden Beispiels näher erläutert.

### Beschichtung von Oberflächen

Die Herstellung einer erfindungsgemäßen Zusammensetzung erfolgt durch Auflösen von 0,1 % (Gew./Vol.) rekombinantem LEA-Protein und 5 % (Gew./Vol.) Trehalose in 100 ml Phosphatpuffer (50 mM Phosphat, 100 mM NaCl, pH 6,8). Anschließend werden 0,02 % (Gew./Vol.) Natriumazid als cytotoxisches Agenz gegen mikrobielle Verunreinigungen zugegeben. Nach Sterilfiltration der erhaltenen Lösung unter Verwendung eines 0,2 µm Filters erfolgt die Aufbewahrung in einer autoklavierten sterilen Flasche.

Zum Beschichten erfindungsgemäß geeigneter Oberflächen werden Biochips (z.B. Objektträger) mit immobilisierten Biomolekülen im Reinraum (bzw. Sterilwerkbank) in die Lösung eingetaucht und mit einer Geschwindigkeit von 2mm/sec wieder aus der Lösung herausgezogen. Dabei trocknet eine dünne Schicht der Trehalose/LEA - Lösung auf dem Chip an und führt zu einer Stabilisierung der Biomoleküle.

Zur Lagerung können die auf diese Weise behandelten Chips mit den stabilisierten Biomolekülen in Kunststofftaschen verpackt und unter Stickstoffatmosphäre bei Raumtemperatur gelagert werden. Die Taschen können vorteilhafterweise in einer lichtdichten Kartonage verpackt werden, um einer möglichen Photodegradation der Proteine vorzubeugen.

Zur Verwendung werden die Chips der Verpackung entnommen und mit Assay-Buffer (z.B. PBS-Puffer) rehydriert (5 min, RT). Anschließend wird die Probenflüssigkeit direkt auf den Chips inkubiert und der Assay durchgeführt. Es können alle Arten der im Stand der Technik bekannten Rezeptor-Liganden Interaktionen durchgeführt werden. Wahlweise kann auch auf das Entfernen des Stabilisators verzichtet werden. In diesem Fall wird die Probe direkt auf die Trehalose-beschichteten Oberflächen gegeben. Dies ist vor allem dann praktikabel, wenn zuvor gezeigt wurde, dass weder Trehalose noch das LEA- Protein mit dem Nachweis interferieren.

## Patentansprüche

1. Verfahren zur Stabilisierung bzw. Konservierung von Biomolekülen, bei dem die Biomoleküle auf Oberflächen immobilisiert sind und mit einer Zusammensetzung beaufschlagt werden, die mindestens ein nicht-reduzierendes Disaccharid und mindestens ein Protein oder Polypeptid der LEA-Klasse umfasst.

2. Verfahren nach Anspruch 1, wobei das nicht-reduzierende Disaccharid ausgewählt wird aus der Gruppe bestehend aus Trehalose (D-Glucopyranosyl-D-glucopyranose) und Saccharose (β-D-Fructofuranosyl-α-D-glycopyranosid).

3. Verfahren nach Anspruch 1 oder 2, wobei das nicht-reduzierende Disaccharid Trehalose ist.

4. Verfahren nach einem der Ansprüche 1 bis 3, wobei das mindestens eine Protein oder Polypeptid der LEA-Klasse ein 11 Aminosäuren umfassendes Motiv aufweist, welches durch die folgende allgemeine Formel (SEQ ID NO. 1) charakterisiert ist:
(1)-(2)-(3)-(4)-(5)-(6)-(7)-(8)-(9)-(10)-E,
wobei
(1) K oder T,
(2) A, G, K, M oder T,
(3) R, D, A, E, Q oder K,
(4) E, K oder S,
(5) T, F, Y oder A,
(6) K, R, T oder A,
(7) D, E oder Q,
(8) S, R, Y oder K,
(9) A oder T, und
(10) G, A oder R,
bedeuten.

5. Verfahren nach einem der vorhergehenden Ansprüche, wobei die Zusammensetzung mindestens ein Protein oder Polypeptid der LEA-Unterklasse 3 mit einer Aminosäuresequenz umfasst, die von einer Nukleotidsequenz kodiert wird, wie sie in der GenBank unter der Zugriffsnummer AF423069 oder S39475 hinterlegt ist.

6. Verfahren nach einem der vorhergehenden Ansprüche, wobei das mindestens eine Protein oder Polypeptid der LEA-Unterklasse 3 ein 11 Aminosäuren umfassendes Motiv aufweist, ausgewählt aus der Gruppe bestehend aus:
(a) K-T-A-E-F-R-D-S-A-G-E (SEQ ID NO. 2),
(b) K-G-Q-E-F-K-E-R-A-G-E (SEQ ID'NO. 3),
(c) K-A-E-E-T-K-Q-R-A-G-E (SEQ ID NO. 4),
(d) K-M-D-E-T-K-Q-R-A-G-E (SEQ ID NO. 5),
(e) K-A-R-K-T-K-D-S-A-A-E (SEQ ID NO. 6),
(f) K-A-K-E-Y-K-D-Y-T-A-E (SEQ ID NO. 7),
(g) K-A-R-E-T-T-E-K-A-R-E (SEQ ID NO. 8), und
(h) T-K-D-S-A-A-E-K-A-R-E (SEQ ID NO. 9).

7. Verfahren nach einem der vorhergehenden Ansprüche, wobei die Zusammensetzung die Komponenten des nicht-reduzierenden Disaccharids und des Proteins oder Polypeptids der LEA-Klasse in jeweiligen Mengen von 0,01 bis 15 beziehungsweise 0,00001 bis 1 Gewichtsprozent, jeweils bezogen auf eine gebrauchsfertige Lösung, umfasst.

8. Verfahren zur Herstellung einer Oberfläche mit immobilisierten und stabilisierten bzw. konservierten Biomolekülen, bei dem die Biomoleküle mit einer Zusammensetzung beaufschlagt werden, die mindestens ein nicht-reduzierendes Disaccharid und mindestens ein Protein oder Polypeptid der LEA-Klasse umfasst.

9. Oberfläche aus einem Material, das aus Glas, Quarzglas, Quarz, Silizium, Polymeren, Nitrozellulose-, Nylon- und Mikrofasermembranen sowie Papier ausgewählt ist, mit immobilisierten und stabilisierten bzw. konservierten Biomolekülen, beaufschlagt mit einer Zusammensetzung, die mindestens ein nicht-reduzierendes Disaccharid und mindestens ein Protein oder Polypeptid der LEA-Klasse umfasst.

10. Oberfläche nach Anspruch 9, wobei die Zusammensetzung die Komponenten des nicht-reduzierenden Disaccharids und des Proteins oder Polypeptids der LEA-Klasse in jeweiligen Mengen von 0,01 bis 15 beziehungsweise 0,00001 bis 1 Gewichtsprozent, jeweils bezogen auf eine gebrauchsfertige Lösung, umfasst.

11. Oberfläche nach Anspruch 9 oder 10, wobei das nicht-reduzierende Disaccharid ausgewählt ist aus der Gruppe bestehend aus Trehalose (D-Glucopyranosyl-D-glucopyranose) und Saccharose (β-D-Fructofuranosyl-α-D-glyco-pyranosid).

12. Oberfläche nach Anspruch 11, wobei das nicht-reduzierende Disaccharid Trehalose ist.

13. Oberfläche nach einem der Ansprüche 9 bis 12, wobei das mindestens eine Protein oder Polypeptid der LEA-Klasse ein 11 Aminosäuren umfassendes Motiv aufweist, welches durch die folgende allgemeine Formel (SEQ. ID NO. 1) charakterisiert ist:
(1)-(2)-(3)-(4)-(5)-(6)-(7)-(8)-(9)-(10)-E,
wobei
(1) K oder T,
(2) A, G, K, M oder T,
(3) R, D, A, E, Q oder K,
(4) E, K oder S,
(5) T, F, Y oder A,
(6) K, R, T oder A,
(7) D, E oder Q,
(8) S, R, Y oder K,
(9) A oder T, und
(10) G, A oder R,
bedeuten.

14. Oberfläche nach einem der Ansprüche 9 bis 13, wobei die Zusammensetzung mindestens ein Protein oder Polypeptid der LEA-Unterklasse 3 mit einer Aminosäuresequenz umfasst, die von einer Nukleotidsequenz kodiert wird, wie sie in der GenBank unter der Zugriffsnummer AF423069 oder S39475 hinterlegt ist.

15. Oberfläche nach einem der Ansprüche 9 bis 14, wobei das mindestens eine Protein oder Polypeptid der LEA-Unterklasse 3 ein 11 Aminosäuren umfassendes Motiv aufweist, ausgewählt aus der Gruppe bestehend aus:
(a) K-T-A-E-F-R-D-S-A-G-E (SEQ ID NO. 2),
(c) K-G-Q-E-F-K-E-R-A-G-E (SEQ ID NO. 3),
(c) K-A-E-E-T-K-Q-R-A-G-E (SEO ID NO. 4),
(d) K-M-D-E-T-K-Q-R-A-G-E (SEQ ID NO. 5),
(e) K-A-R-K-T-K-D-S-A-A-E (SEQ ID NO. 6),
(f) K-A-K-E-Y-K-D-Y-T-A-E (SEQ ID NO. 7),
(g) K-A-R-E-T-T-E-K-A-R-E (SEQ ID NO. 8), und
(h) T-K-D-S-A-A-E-K-A-R-E (SEQ ID NO. 9).

16. Analytische und/oder diagnostische Vorrichtung, umfassend eine Oberfläche nach einem der Ansprüche 9 bis 15.

17. Vorrichtung nach Anspruch 16, ausgewählt aus der Gruppe bestehend aus Biochips, Sensorchips, Mikrotiterplatten, Reaktionsröhrchen und Kulturschalen.

18. Verwendung einer Oberfläche nach einem der Ansprüche 9 bis 15 mit immobilisierten und stabilisierten bzw. konservierten Biomolekülen, beaufschlagt mit einer Zusammensetzung, die mindestens ein nicht-reduzierendes Disaccharid und mindestens ein Protein oder Polypeptid der LEA-Klasse umfasst, für analytische und/oder diagnostische Zwecke in vitro.

## Claims

1. Method for stabilization or preservation of biomolecules, wherein the biomolecules are immobilized on surfaces and are covered with a composition which comprises at least one non-reducing disaccharide and at least one protein or polypeptide of the LEA-class.

2. Method according to claim 1, wherein the non-reducing disaccharide is selected from the group consisting of trehalose (D-glucopyranosyl-D-glucopyranose) and sucrose (β-D-fructofuranosyl-α-D-glycopyranoside).

3. Method according to claim 1 or 2, wherein the non-reducing disaccharide is trehalose.

4. Method according to one of claims 1 to 3, wherein the at least one protein or polypeptide of the LEA-class has a motif of 11 amino acids which is **characterized by** the following general formula (SEQ ID NO.1) :
(1)-(2)-(3)-(4)-(5)-(6)-(7)-(8)-(9)-(10)-E,
wherein
(1) stands for K or T,
(2) stands for A, G, K, M or T,
(3) stands for R, D, A, E, Q or K,
(4) stands for E, K or S,
(5) stands for T, F, Y or A,
(6) stands for K, R, T or A,
(7) stands for D, E or Q,
(8) stands for S, R, Y or K,
(9) stands for A or T, and
(10) stands for G, A or R.

5. Method according to one of the preceding claims, wherein the composition comprises at least one protein or polypeptide of the LEA-subclass 3 with an amino acid sequence which is encoded by a nucleotide sequence as is deposited in GenBank under the access number AF423069 or S39475.

6. Method according to one of the preceding claims, wherein the at least one protein or polypeptide of the LEA-subclass 3 has a motif of 11 amino acids selected from the group consisting of:
(a) K-T-A-E-F-R-D-S-A-G-E (SEQ ID NO. 2),
(b) K-G-Q-E-F-K-E-R-A-G-E (SEQ ID NO. 3),
(c) K-A-E-E-T-K-Q-R-A-G-E (SEQ ID NO. 4),
(d) K-M-D-E-T-K-Q-R-A-G-E (SEQ ID NO. 5),
(e) K-A-R-K-T-K-D-S-A-A-E (SEQ ID NO. 6),
(f) K-A-K-E-Y-K-D-Y-T-A-E (SEQ ID NO. 7),
(g) K-A-R-E-T-T-E-K-A-R-E (SEQ ID NO. 8), and
(h) T-K-D-S-A-A-E-K-A-R-E (SEQ ID NO. 9).

7. Method according to one of the preceding claims, wherein the composition comprises the components of the non-reducing disaccharide and the protein or polypeptide of the LEA-class in respective quantities of 0.01 to 15 and 0.00001 to 1 weight percent, each referring to a ready-to-use solution.

8. Method for the production of a surface with immobilized and stabilized or preserved biomolecules, wherein the biomolecules are covered with a composition which comprises at least one non-reducing disaccharide and at least one protein or polypeptide of the LEA-class.

9. Surface of a material which is selected from glass, silica glass, silica, silicon, polymers, nitrocellulose-, nylon-and microfiber membranes as well as paper, with immobilized and stabilized or preserved biomolecules covered with a composition which comprises at least one non-reducing disaccharide and at least one protein or polypeptide of the LEA-class.

10. Surface according to claim 9, wherein the composition comprises the components of the non-reducing disaccharide and the protein or polypeptide of the LEA-class in respective quantities of 0.01 to 15 weight percent and 0.00001 to 1 weight percent each referring to a ready-to-use solution.

11. Surface according to claim 9 or 10, wherein the non-reducing disaccharide is selected from the group consisting of trehalose (D-glucopyranosyl-D-glucopyranose) and sucrose (β-D-fructofuranosyl-α-D-glycopyranoside).

12. Surface according to claim 11, wherein the non-reducing disaccharide is trehalose.

13. Surface according to one of claims 9 to 12, wherein the at least one protein or polypeptide of the LEA-class has a motif of 11 amino acids which is **characterized by** the following general formula (SEQ ID NO.1):
(1)-(2)-(3)-(4)-(5)-(6)-(7)-(8)-(9)-(10)-E,
wherein
(1) stands for K or T,
(2) stands for A, G, K, M or T,
(3) stands for R, D, A, E, Q or K,
(4) stands for E, K or S,
(5) stands for T, F, Y or A,
(6) stands for K, R, T or A,
(7) stands for D, E or Q,
(8) stands for S, R, Y or K,
(9) stands for A or T, and
(10) stands for G, A or R.

14. Surface according to one of claims 9 to 13, wherein the composition comprises at least one protein or polypeptide of the LEA-subclass 3 with an amino acid sequence which is encoded by a nucleotide sequence as deposited in GenBank under the access number AF423069 or S39475.

15. Surface according to one of claims 9 to 14, wherein the at least one protein or polypeptide of the LEA-subclass 3 has a motif comprising 11 amino acids selected from the group consisting of:
(a) K-T-A-E-F-R-D-S-A-G-E (SEQ ID NO. 2),
(c) K-G-Q-E-F-K-E-R-A-G-E (SEQ ID NO. 3),
(c) K-A-E-E-T-K-Q-R-A-G-E (SEQ ID NO. 4),
(d) K-M-D-E-T-K-Q-R-A-G-E (SEQ ID NO. 5),
(e) K-A-R-K-T-K-D-S-A-A-E (SEQ ID NO. 6),
(f) K-A-K-E-Y-K-D-Y-T-A-E (SEQ ID NO. 7),
(g) K-A-R-E-T-T-E-K-A-R-E (SEQ ID NO. 8), and
(h) T-K-D-S-A-A-E-K-A-R-E (SEQ ID NO. 9).

16. Analytical and/or diagnostic device comprising a surface according to one of claims 9 to 15.

17. Device according to claim 16 selected from the group consisting of biochips, sensor chips, microtiter plates, reaction tubes and culture plates.

18. Use of a surface according to one of claims 9 to 15 with immobilized and stabilized or preserved biomolecules covered with a composition which comprises at least one non-reducing disaccharide and at least one protein or polypeptide of the LEA-class, for analytical and/or diagnostic purposes *in vitro.*

## Revendications

1. Procédé pour la stabilisation, respectivement la conservation, de biomolécules dans lequel les biomolécules sont immobilisées sur des surfaces et sont recouvertes avec une composition qui contient au moins un disaccharide non-réducteur et au moins une protéine ou polypeptide de la classe LEA.

2. Procédé selon la revendication 1 où le disaccharide non-réducteur est choisi dans un groupe constitué du tréhalose (D-glucopyranosyl-D-glucopyranose) et du sucrose (β-D-fructofuranosyl-α-D-glycopyranoside).

3. Procédé selon la revendication 1 ou 2 où le disaccharide non-réducteur est le tréhalose.

4. Procédé selon une des revendications 1 à 3 dans lequel la protéine ou polypeptide de la classe LEA, contenue au minimum dans la composition, présente un motif composé de 11 acides aminés qui est **caractérisé par** la formule générale suivante (SEQ ID No.1) :
(1)-(2)-(3)-(4)-(5)-(6)-(7)-(8)-(9)-(10)-E,
dans laquelle
(1) désigne K ou T,
(2) désigne A, G, K, M, ou T,
(3) désigne R, D, A, E, Q ou K,
(4) désigne E, K ou S,
(5) désigne T, F, Y ou A,
(6) désigne K, R, T ou A,
(7) désigne D, E ou Q,
(8) désigne S, R, Y ou K,
(9) désigne A ou T, et
(10) désigne G, A ou R.

5. Procédé selon une des revendications précédentes dans lequel la composition contient au moins une protéine ou polypeptide de la sous-classe des LEA 3, avec une séquence d'acides aminés qui est codée par une séquence nucléotidique comme elle est déposée dans la banque de donnée GenBank sous les numéros d'accession AF423069 ou S39475.

6. Procédé selon une des revendications précédentes dans lequel la protéine ou polypeptide de la sous-classe des LEA 3 qui est au minimum contenue dans la composition, présente un motif contenant 11 acides aminés, choisi dans un groupe composé de :
(a) K-T-A-E-F-R-D-S-A-G-E (SEQ ID No. 2),
(b) K-G-Q-E-F-K-E-R-A-G-E (SEQ ID No. 3),
(c) K-A-E-E-T-K-Q-R-A-G-E (SEQ ID No. 4),
(d) K-M-D-E-T-K-Q-R-A-G-E (SEQ ID No. 5),
(e) K-A-R-K-T-K-D-S-A-A-E (SEQ ID No. 6),
(f) K-A-K-E-Y-K-D-Y-T-A-E (SEQ ID No. 7),
(g) K-A-R-E-T-T-E-K-A-R-E (SEQ ID No. 8),
(h) T-K-D-S-A-A-E-K-A-R-E (SEQ ID No. 9),

7. Procédé selon une des revendications précédentes dans lequel le mélange des composants contient des quantités respectives de 0,01 à 15 pour cent/poids de disaccharides non-réducteurs et 0,00001 à 1 pour cent/poids de protéines ou polypeptides de la classe LEA, respectivement relatives à la solution prête à l'emploi.

8. Procédé pour la production d'une surface avec des biomolécules immobilisées et stabilisées, respectivement conservées, dans lequel les biomolécules sont recouvertes avec une composition qui contient au moins un disaccharide non-réducteur et au moins une protéine ou polypeptide de la classe LEA.

9. Surfaces composée d'un matériel, choisi parmi le verre, le verre de quartz, le quartz, le silicium, les polymères, les membranes de nitrocellulose, de nylon et de microfibre ainsi que de papier, avec des biomolécules immobilisées et stabilisées, respectivement conservées, recouvertes avec une composition qui contient au moins un disaccharide non-réducteur et au moins une protéine ou polypeptide de la classe LEA.

10. Surfaces selon la revendication 9, pour lesquelles le mélange des composants contient des quantités respectives de 0,01 à 15 pour cent/poids de disaccharides non-réducteurs et 0,00001 à 1 pour cent/poids de protéines ou polypeptides de la classe LEA, respectivement relatives à la solution prête à l'emploi.

11. Surfaces selon la revendication 9 ou 10, pour lesquelles le disaccharide non-réducteur est choisi dans le groupe composé du tréhalose (D-glucopyronasyl-D-glucopyranose) et du sucrose (β-D-fructofuranosyl-α-D-glycopyranoside).

12. Surfaces selon la revendication 11 pour lesquelles le disaccharide non-réducteur est le tréhalose.

13. Surfaces selon une des revendications 9 à 12 pour lesquelles la protéine ou polypeptide de la classe LEA, contenue au minimum, présente un motif composé de 11 acides aminés qui est **caractérisé par** la formule générale suivante (SEQ ID No.1) :
(1)-(2)-(3)-(4)-(5)-(6)-(7)-(8)-(9)-(10)-E,
dans laquelle
(1) désigne K ou T,
(2) désigne A, G, K, M, ou T,
(3) désigne R, D, A, E, Q ou K,
(4) désigne E, K ou S,
(5) désigne T, F, Y ou A,
(6) désigne K, R, T ou A,
(7) désigne D, E ou Q,
(8) désigne S, R, Y ou K,
(9) désigne A ou T, et
(10) désigne G, A ou R.

14. Surfaces selon une des revendications 9 à 13 pour lesquelles la composition contient au moins une protéine ou polypeptide de la sous-classe des LEA 3, avec une séquence d'acides aminés qui est codée par une séquence nucléotidique comme elle est déposée dans la banque de donnée GenBank sous les numéros d'accession AF423069 ou S39475.

15. Surfaces selon une des revendications 9 à 14 pour lesquelles la protéine ou polypeptide de la sous-classe des LEA 3 qui est au minimum contenue dans la composition, présente un motif contenant 11 acides aminés, choisi dans un groupe composé de :
(a) K-T-A-E-F-R-D-S-A-G-E (SEQ ID No. 2),
(b) K-G-Q-E-F-K-E-R-A-G-E (SEQ ID No. 3),
(c) K-A-E-E-T-K-Q-R-A-G-E (SEQ ID No. 4),
(d) K-M-D-E-T-K-Q-R-A-G-E (SEQ ID No. 5),
(e) K-A-R-K-T-K-D-S-A-A-E (SEQ ID No. 6),
(f) K-A-K-E-Y-K-D-Y-T-A-E (SEQ ID No. 7),
(g) K-A-R-E-T-T-E-K-A-R-E (SEQ ID No. 8),
(h) T-K-D-S-A-A-E-K-A-R-E (SEQ ID No. 9),

16. Dispositif analytique et/ou diagnostique contenant une surface selon une des revendications 9 à 15.

17. Dispositif selon la revendication 16 choisi dans un groupe composé de biopuces, de puces sensorielles, de plaques de microtitration, de tubes de réaction et de boîtes de cultures.

18. Utilisation d'une surface selon une des revendications 9 à 15, avec des biomolécules immobilisées et stabilisées, respectivement conservées, recouverte avec une composition qui contient au moins un disaccharide non-réducteur et au moins une protéine ou polypeptide de la classe LEA, pour des applications analytiques et/ou diagnostiques in vitro.
